Europäisches Patentamt

European Patent Office

Office européen des brevets

(19)

(11) Veröffentlichungsnummer : **0 336 231 B1**

(12) **EUROPÄISCHE PATENTSCHRIFT**

(45) Veröffentlichungstag der Patentschrift :
**07.09.94 Patentblatt 94/36**

(51) Int. Cl.$^5$ : **G01N 33/53**, G01N 33/543

(21) Anmeldenummer : **89105232.6**

(22) Anmeldetag : **23.03.89**

(54) **Verfahren zur Stabilisierung von biologisch aktiven Substanzen in immobilisierter Form.**

(30) Priorität : **07.04.88 DE 3811659**

(43) Veröffentlichungstag der Anmeldung :
**11.10.89 Patentblatt 89/41**

(45) Bekanntmachung des Hinweises auf die
Patenterteilung :
**07.09.94 Patentblatt 94/36**

(84) Benannte Vertragsstaaten :
**AT BE CH DE ES FR GB GR IT LI LU NL SE**

(56) Entgegenhaltungen :
**EP-A- 0 140 489**
**EP-A- 0 212 522**

(56) Entgegenhaltungen :
**WO-A-86/04095**
**DE-A- 2 146 597**
**DE-A- 3 225 250**
**US-A- 2 172 357**

(73) Patentinhaber : **Henning Berlin Anlagen GmbH**
**Komturstrasse 58-62**
**D-12099 Berlin (DE)**

(72) Erfinder : **Beier, Wilfried, Dr.**
**Im Erpelgrund 65**
**D-1000 Berlin 27 (DE)**

(74) Vertreter : **Andrae, Steffen, Dr. et al**
**Balanstrasse 55**
**D-81541 München (DE)**

EP 0 336 231 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die Erfindung betrifft ein Verfahren zur Stabilisierung von biologisch aktiven Substanzen in immobilisierter Form, bei dem die immobilisierten biologisch aktiven Substanzen in Gegenwart eines inerten Füllstoffs getrocknet werden. Derartige Verfahren sind im Bereich der Biochemie, der Biotechnologie und der Immundiagnostik von großer Bedeutung.

Unter "biologisch aktive Substanzen" sind dabei insbesondere Enzyme, Enzyminhibitoren, Antigene, Antikörper, Organellen und ganze Zellen sowie Bestandteile derartiger Substanzen zu verstehen, wobei diese biologisch aktiven Substanzen überwiegend proteinischer Natur sind.

Wenn derartige biologisch aktive Substanzen in eine immobilisierte Form überführt werden, lassen sich ihre funktionellen Eigenschaften für die Durchführung heterogener Reaktionen in der Festphasenbiochemie ausnutzen. Derartige Reaktionen sind beispielsweise Substratumsetzungen mit Hilfe immobilisierter Enzyme, Organellen oder ganzer Zellen sowie insbesondere auch heterogene Immunoassays, die auf der Durchführung immunologischer Reaktionen mit Hilfe immobilisierter Antikörper bzw. Antigene beruhen.

Die Immobilisierung der biologisch aktiven Substanzen erfolgt dabei auf festen Trägermaterialien verschiedener Art, wobei insbesondere eine Immobilisierung an polymeren Trägermaterialien oder Glasoberflächen durchgeführt wird. Die Immobilisierung an polymeren Trägern erfolgt dabei je nach der Natur des Trägermaterials und der zu immobilisierenden Substanz durch physikalische Adsorption auf den Oberflächen der natürlichen oder synthetisch hergestellten festen Trägermaterialien, durch kovalente Bindung über funktionelle reaktive Gruppen oder durch Einschlußfixierung in die Poren vernetzter Gele.

Damit die funktionellen Eigenschaften der immobilisierten biologisch aktiven Trägermaterialien im gewünschten Sinne ausgenutzt werden können, ist es Voraussetzung, daß bei der Immobilisierung die biologische Aktivität nicht oder nur unwesentlich beeinträchtigt wird. Um eine derartige Beeinträchtigung zu vermeiden, müssen die jeweiligen Immobilisierungsverfahren und die Reaktionsbedingungen bei der Immobilisierung in geeigneter Weise gewählt werden.

Insbesondere dann, wenn die immobilisierten biologisch aktiven Substanzen nicht sofort in die beabsichtigten heterogenen Reaktionen eingesetzt werden können, und das ist bei allen Handelsprodukten der Fall, ist nicht nur die Erhaltung der biologischen Aktivität direkt bei der Immobilisierung von Bedeutung, sondern diese biologische Aktivität der Immobilisate muß über einen langen Zeitraum, der sich von einigen Monaten bis zu einigen Jahren erstrecken kann, erhalten bleiben, und zwar vorzugsweise unter Bedingungen, unter denen derartige Immobilisate gut aufbewahrt werden können.

Die Möglichkeit einer Aufbewahrung der Immobilisierungsprodukte in einem trockenen Zustand weist dabei gegenüber der Aufbewahrung im feuchten Zustand zahlreiche evidente Vorteile auf. In vielen Fällen ist die Bereitstellung der Immobilisierungsprodukte in trockenem Zustand sogar eine unabdingbare Voraussetzung für deren praktische Nutzbarmachung.

Zur Gewinnung trockener Produkte muß sich daher an die Immobilisierung ein Trocknungsschritt anschließen, für den einfache Lufttrocknung, Trocknung im Vakuum oder Gefriertrocknung zur Auswahl stehen. Es hat sich nunmehr jedoch gezeigt, daß eine einfache Trocknung von immobilisierten biologisch aktiven Substanzen sehr häufig zu einem vollständigen oder teilweisen Verlust der biologischen Wirksamkeit führt, der insbesondere nach längerer Aufbewahrungszeit spürbar wird.

Der Grad des Verlusts der biologischen Wirksamkeit hängt dabei von den Eigenschaften und der Natur der immobilisierten biologisch aktiven Substanz, von den Eigenschaften des zur Immobilisierung verwendeten Trägermaterials sowie von der Art der Trocknung ab. Immobilisierte Antikörper büßen dabei sehr häufig während einer Aufbewahrung im trockenen Zustand einen Teil ihrer immunologischen Eigenschaften ein, so daß die bestimmungsgemäße Verwendung derartiger immobilisierter Antikörper durch einen mit der Dauer der Aufbewahrung zunehmenden Verlust an Sensitivität und Präzision in der Analyse eingeschränkt wird.

Es ist nunmehr bereits bekannt, daß die Verluste an Wirksamkeit der biologisch aktiven Substanzen bei der Trocknung dadurch vermindert oder sogar ganz vermieden werden können, wenn man die Trocknung unter geeigneten Bedingungen in Gegenwart von Füllstoffen durchführt. So ist es aus der US-PS 3 133 001 bekannt, daß Aktivitätsverluste bei der Trocknung von Enzymen vermieden werden können, wenn den Lösungen der Enzyme vor der Trocknung, insbesondere der Gefriertrocknung, als Füllstoffe wirkende Disaccharide zugesetzt werden.

Aus der DE-PS 29 52 815 ist es ferner bekannt, daß die Trocknung in Gegenwart von Füllstoffen auch bei den noch kritischeren immobilisierten biologisch aktiven Substanzen (Proteinmaterialien) den Aktivitätsverlust vermindert. Die DE-PS 29 52 815 betrifft dabei die Gefriertrocknung von Suspensionen von an teilchenförmigen Trägermaterialien immobilisierten Eiweißstoffen in Gegenwart von inerten wasserlöslichen festen Füllmitteln, wobei als derartige Füllmittel anorganische Salze, Kohlenhydrate, Proteine oder synthetische Polymere, insbesondere feste Polyalkylenglycole, genannt sind. In der GB-Patentanmeldung 2 124 231 ist die Sta-

2

bilisierung von an feste Träger gebundenen Antikörpern oder Antigenen durch Überziehen mit immunologisch inerten Proteinen wie beispielsweise Serumalbumin, Gelatine und abgebaute Gelatine beschrieben.

Es hat sich nunmehr jedoch gezeigt, daß der Stabilisierungseffekt der bisher in diesem Zusammenhang diskutierten Substanzen unzureichend war, insbesondere im Falle empfindlicher Antikörper und Antigene, die im Bereich der Immundiagnostik von Schilddrüsenzuständen Verwendung finden.

Es ist somit Aufgabe der vorliegenden Erfindung, ein Verfahren zur Stabilisierung von biologisch aktiven Substanzen in immobilisierter Form, bei dem die immobilisierten biologisch aktiven Substanzen in Gegenwart eines inerten Füllstoffs getrocknet werden, zu schaffen, das im Hinblick auf eine Verhinderung von Aktivitätsverlusten selbst bei längerer Lagerdauer wirksamer ist als die vorbekannten Verfahren.

Diese Aufgabe wird bei einem Verfahren der genannten Art dadurch gelöst, daß das im Patentanspruch 1 definierte Gemisch aus einem Zuckeralkohol und einem kristallisationsverzögernd wirkenden Anteil an hydrierten Oligosacchariden oder Zuckeralkohol-Derivaten als Füllstoff verwendet wird.

Vorteilhafte Ausgestaltungen des erfindungsgemäßen Verfahrens sind den Unteransprüchen zu entnehmen.

Bei dem erfindungsgemäßen Verfahren werden die zu trocknenden Immobilisierungsprodukte, insbesondere für die immunologische in-vitro-Analyse bestimmte immobilisierte Antikörper und Antigene, vor ihrer Trocknung mit einer wäßrigen oder gepufferten Lösung eines kristallisationsverzögerten Gemischs, das als Hauptbestandteil einen niedermolekularen geradkettigen Zuckeralkohol enthält, behandelt.

Die als Kristallisationsverzögerer anwesenden Substanzen sind chemisch den Zuckeralkoholen verwandt und sind insbesondere hydrierte Oligosaccharide, können beispielsweise aber auch Zuckeralkohol-Derivate vom in der US-PS 2 172 357 erwähnten Typ sein.

Der Begriff "Zuckeralkohol-Derivate" umfaßt somit insbesondere verzweigtkettige Desoxyhexite und -pentite und Anhydroderivate der Zuckeralkohole, wobei Sorbitane, insbesondere 1,4-Sorbitane, bevorzugte Zuckeralkohol-Derivate sind.

Eine besonders gute stabilisierende Wirkung wurde bei der Verwendung einer wäßrigen Lösung einer Mischung aus D-Sorbit mit hydrierten Oligosacchariden, die durch Hydrierung von Glucosesirup als Ausgangsmaterial erzeugt wurde, erhalten. Der Anteil des D-Sorbits am gesamten Feststoffgehalt einer solchen wäßrigen Lösung liegt dabei im Bereich von 50 bis 95 Gew.-%, vorzugsweise von 70 bis 90 Gew.-%. Der Anteil an hydrierten Oligosacchariden, der die gewünschte Kristallisationsverzögerung bewirkt, liegt, bezogen auf den Feststoffgehalt, üblicherweise im Bereich von 1 bis 6 Gew.-%. Das Gemisch kann neben D-Sorbit in geringeren Mengen auch noch andere Substanzen von verwandter chemischer Natur enthalten, wobei insbesondere Mannit zu nennen ist, dessen Anteil bis zu 8 % betragen kann. Die durch Trocknung derartiger Lösungen erzeugten Zuckeralkohol-Gemische zeigen, wenn überhaupt, erst bei ungewöhnlich niedrigen Temperaturen Kristallisationserscheinungen. Der Grad der Kristallisation kann dabei leicht über den Anteil an hydrierten Oligosacchariden im Gemisch beeinflußt werden. Bestimmte, für die Zwecke des erfindungsgemäßen Verfahrens geeignete Gemische aus D-Sorbit und hydrierten Oligosacchariden sind dabei als Handelsprodukte erhältlich (Karion (Wz) F und FP der Firma Merck).

Es ist bekannt, daß stark hygroskopische Substanzen, wie beispielsweise Glycerin und Glycole, in feuchter Umgebung Wasser sehr schnell aufnehmen und in trockener Umgebung sehr schnell wieder abgeben. Bei biologisch aktiven Substanzen führen derartige Schwankungen des Feuchtigkeitsgrades zu Aktivitätsverlusten. Die erfindungsgemäß zu verwendenden Zuckeralkohol-Gemische stellen dagegen ein Mittel dar, das aufgrund seiner relativ geringen Hygroskopizität den Feuchtigkeitsgrad von immobilisierten Proteinen in besonderer Weise unabhängig von der Feuchtigkeit der Umgebung reguliert und damit eine proteinstabilisierende Wirkung entwickelt.

Wie nachfolgend anhand von Vergleichen mit als Stabilisatoren diskutierten Füllmitteln nachgewiesen wird, ist die durch die erfindungsgemäß zu verwendenden Gemische erzielte Stabilisierung erheblich besser als eine Stabilisierung mit bekannten Füllmitteln, auch wenn diese in ihrer chemischen Natur relativ ähnlich sind.

Die erfolgreiche Stabilisierung ist grade für immundiagnostische in-vitro-Analysen, wie Radio-, Enzym-, Fluoreszenz- und Lumineszenz-Immunoassays von höchster Bedeutung, wenn diese schnell und sicher durchgeführt werden sollen, da in diesen Fällen die an Festkörper gebundenen Antikörper bzw. Antigene über einen langen Zeitraum stabil sein müssen. Die Festkörper, die in Immunoassays Verwendung finden, können mikropolymere Partikel oder magnetisierbare Partikel mit einem Korndurchmesser von etwa 1 µm sein, oder sie können bis zu 6 mm große Kunststoff-Kugeln sein. Aus praktischen Gründen besonders bevorzugt sind die Festkörper jedoch Mikrotiterplatten oder Röhrchen aus Polystyrol, Polypropylen, Polyamid, Polyethylen und ähnlichen Kunststoffen. Die Vorteile des erfindungsgemäßen Verfahrens zeigen sich in diesen Fällen besonders ausgeprägt, nämlich wenn Antikörper oder Antigene adsorptiv oder kovalent an Mikrotiterplatten oder an die inneren Oberflächen von Röhrchen gebunden sind, d. h. wenn die in der Immundiagnostik unter dem Namen Coated tube-Technik bekannte Technik gewählt werden soll.

Ein spezieller, in den Beispielen noch näher erläuterter Anwendungsfall betrifft die Immobilisierung von monoklonalen oder polyklonalen anti-human Thyreotropin (hTSH)-Antikörpern an den inneren Oberflächen von Polystyrol-Röhrchen, die zuvor durch chemische und/oder physikalische Behandlung zur Adsorptionsimmobilisierung vorbereitet worden sein können, mit deren Hilfe sich in der immundiagnostischen in-vitro-Analyse die TSH-Konzentrationen in Humansera bestimmen lassen. Es hat sich jedoch gezeigt, daß die hervorragenden Stabilisierungseigenschaften der erfindungsgemäß zu verwendenden Gemische sich auch bei anderen Antikörpern und Antigenen zeigen, wobei insbesondere die auf dem engeren Interessengebiet der Anmelderin liegenden Substanzen wie anti-T3-Antikörper, anti-T4-Antikörper, anti-PTH-Antikörper und anti-Thyreoglobulin-Antikörper sowie T4- und T3-Antigene geprüft wurden.

Das erfindungsgemäße Verfahren wird in der Praxis bequemerweise so durchgeführt, daß die bei der Immobilisierung erhaltenen Produkte vor der Trocknung ein- oder mehrmals mit der Lösung des Zuckeralkohol-Gemischs manuell oder mit Hilfe von Automaten gewaschen oder einfach in eine solche Lösung getaucht werden. Die notwendige Einwirkungszeit wird bestimmt durch die Art der jeweiligen biologisch aktiven Substanz sowie auch durch die Art und die Konzentration der Behandlungslösung. Die notwendige Einwirkzeit kann dabei von wenigen Minuten bis zu einigen Stunden liegen und anhand einfacher Vorversuche ohne Schwierigkeiten ermittelt werden. Die zur Erreichung des gewünschten Stabilisierungseffektes bei den Immobilisierungsprodukten üblichen Einwirkungszeiten liegen bei 30 bis 120 min.

Das Gemisch aus Zuckeralkohl und kristallisationsverzögernd wirkenden hydrierten Oligosacchariden bildet bei dem anschließenden Trockenvorgang auf dem festen, insbesondere polymeren Trägermaterial eine homogene und feste, nicht durch kristallisierende Bereiche unterbrochene oder poröse Schutzschicht aus und unterscheidet sich in dieser Eigenschaft von anderen bekannten Stabilisatoren. Aufgabe der Schutzschicht ist es, durch einen chemisch-physikalisch und mechanisch wirkenden Stabilisierungseffekt die biologische Wirksamkeit der Immobilisierungsprodukte über einen langen Zeitraum zu gewährleisten. Der chemisch-physikalische Schutzeffekt kann durch die Ausbildung von Wasserstoffbrücken, durch die Unterstützung von van-der-Waals-Bindungen sowie als eine Fixierung der Molekülgeometrie der biologisch wirksamen Substanz gedeutet werden. Der mechanisch wirksame Stabilisierungseffekt des gebildeten festen Films aus dem erfindungsgemäß zu verwendenden Zuckeralkohl-Gemisch betrifft beispielsweise die Regulierung des Feuchtigkeitsgehalts der immobilisierten Substanz. Außerdem verhindert der feste Film eine nachträgliche, durch die lange Aufbewahrungszeit an sich begünstigte mikrobielle Kontamination.

Die Schichtdicke der Schutzschicht wird für ein bestimmtes Immobilisierungsprodukt für den konkreten Fall in Vorversuchen festgelegt. Sie hängt von der Art und den Eigenschaften des Zuckeralkohols, der biologisch aktiven Substanz, dem zur Immobilisierungsreaktion verwendeten Trägermaterial und der beabsichtigten Verwendungsart ab. Die Feststoffkonzentration des Gemischs aus niedermolekularem Zuckeralkohol und hydrierten Oligosacchariden in der wäßrigen oder gepufferten wäßrigen Lösung kann in jedem Einzelfall unter Berücksichtigung des Stabilisierungseffekts und des optischen Aussehens der Immobilisate variiert werden. Als besonders günstig haben sich dabei die bevorzugten Feststoff-Konzentrationen von 0,1 bis 10 Gew.-%, insbesondere von 1 bis 5 Gew.-% erwiesen. Eine für die meisten Anwendungszwecke geeignete vorzugsweise Feststoffkonzentration beträgt 3 Gew.-%.

Es kann sich im Einzelfall als zweckmäßig erweisen, zusätzlich zu dem erfindungsgemäß zu verwendenden Gemisch weitere die Stabilisierung der biologisch aktiven Substanz fördernde und unterstützende Füllstoff-Substanzen zuzusetzen.

Nach der Behandlung der auf den Trägermaterialien immobilisierten biologisch aktiven Substanzen mit einer wäßrigen Lösung des erfindungsgemäß zu verwendenden Gemischs erfolgt eine Trocknung an der Luft, im Vakuum oder, vorzugsweise, durch Gefriertrocknung.

Die biologische Aktivität der biologisch aktiven Substanzen wird durch die Gefriertrocknung der Immobilisate in Gegenwart des erfindungsgemäß zu verwendenden Gemischs nicht merklich vermindert.

Die sich beim Trocknen bildende Schutzschicht besteht aus den festen Bestandteilen der eingesetzten Behandlungslösung und bewahrt die biologische Wirksamkeit über einen langen Zeitraum.

Die gebildete Schutzschicht besteht aus einem klaren, durchsichtigen, farblosen Filmüberzug und beeinträchtigt in keiner Weise das optische Aussehen der Immobilisierungsprodukte. Die Schutzschicht löst sich sofort ohne weiteres auf, ohne daß gerührt oder geschüttelt werden muß, wenn die Immobilisierungsprodukte bestimmungsgemäß mit einer wäßrigen Lösung oder mit Körperflüssigkeiten wie Blut, Plasma und Serum in Kontakt gebracht werden. Im Falle der immundiagnostischen in-vitro-Analyse stören die Bestandteile des Gemischs aus Zuckeralkohol und hydrierten Oligosacchariden in keiner Weise die durchzuführenden Bestimmungen. Ein der eigentlichen immundiagnostischen in-vitro-Analyse vorausgehender zusätzlicher, das Testverfahren erschwerender Schritt zur Eliminierung der Bestandteile des Füllstoffs ist somit nicht notwendig.

Enthält das zu trocknende Immobilisierungsprodukt keinen Zusatz eines nicht kristallisierenden, niedermolekularen Zuckeralkohols, so nimmt die biologische Wirksamkeit bei der Aufbewahrung des Immobilisats in trockenem Zustand schon nach sehr kurzer Zeit zunehmend ab, wodurch die Eignung der Immobilisierungsprodukte für die gewünschte Verwendung eingeschränkt wird.

Unter der bei der Trocknung gebildeten homogenen Schutzschicht, vorzugsweise unter der Schutzschicht aus D-Sorbit und hydrierten Oligosacchariden, können die Immobilisierungsprodukte einen langen Zeitraum bis zu ihrer Verwendung ohne Aktivitätsverlust gelagert werden.

Somit gestattet es die Erfindung, auf anorganische Träger, wie Glas, auf vollsynthetische polymere Träger, beispielsweise auf der Basis von Polystyrol, Polyvinylchlorid, Polyamid, Polyethylen, auf natürliche polymere Träger, beispielsweise auf Dextrane oder Alginate, kovalent oder adsorptiv immobilisierte biologisch aktive Substanzen im trockenen Zustand über einen Zeitraum von einigen Monaten bis zu einigen Jahren stabil zu erhalten.

Dadurch wird die industrielle Anwendungsmöglichkeit biologisch aktiver Substanzen, d.h. die Schaffung von Handelsprodukten wie Kits für die Immundiagnostik, insbesondere Coated-tube-Kits, erheblich erleichtert.

Nachfolgend wird die Erfindung anhand von konkreten Ausführungsbeispielen und Vergleichsbeispielen noch näher erläutert.

Beispiel 1

a) Immobilisierung von anti-hTSH-Antikörpern durch Adsorption auf Polystyrol-Röhrchen

9,68 g Trishydroxymethylaminomethan und 4,64 g Natriumchlorid werden in 8 l destilliertem Wasser gelöst, und die Lösung wird mit verdünnter Salzsäure auf pH 7,8 titriert.

In der Pufferlösung werden 20 mg eines monoklonalen anti-hTSH-Antikörpers gelöst, und die Lösung wird 30 min unter Rühren oder Schütteln equilibriert.

Die Antikörper-Lösung wird in Portionen von 300 µl pro Röhrchen mittels eines Pipettierautomaten auf 22000 PolystyrolRöhrchen verteilt.

Die Immobilisierung der Antikörper auf den Oberflächen der Polystyrol-Röhrchen erfolgt durch physikalische Adsorption innerhalb von 20 h bei Raumtemperatur oder bei 4 bis 8°C. Anschließend wird überschüssiger Antikörper durch einfaches Waschen mit Wasser entfernt.

b) Stabilisierung der unter a) immobilisierten anti-hTSH-Antikörper und Trocknung der beschichteten Röhrchen

4,5 kg eines Gemischs aus D-Sorbit und hydrierten Oligosacchariden (Handelsprodukt Karion (Wz) F), 750 g Bovines Serumalbumin BSA und 7,5 g Natriumazid werden in 150 l destilliertem Wasser gelöst.

Mit dieser Lösung werden die Röhrchen befüllt, und die Lösung wir 2 h in den Röhrchen gelassen. Anschließend wird die Lösung dekantiert, und die Röhrchen werden durch Lyophilisation getrocknet.

Bis zu ihrer Verwendung werden die Röhrchen bei 4 bis 8°C oder bei Raumtemperatur ohne weitere Schutzmaßnahmen vor denaturierenden Einflüssen gelagert.

c) Charakterisierung der immobilisierten und stabilisierten anti-hTSH-Antikörper

Die mit der Stabilisierungslösung behandelten immobilisierten anti-hTSH-Antikörper werden in einem immunoradiometrischen Assay hinsichtlich ihres Antigen-Bindungsverhaltens und ihrer Präzision charakterisiert.

Die Charakterisierung erfolgt mit einem zweiten J-125-markierten anti-TSH-Antikörper in Gegenwart einer vorgegebenen Menge bis zu 80 mU/l TSH in Humanserum. Nach einer 2stündigen Inkubation der immobilisierten und stabilisierten anti-hTSH-Antikörper mit 200 µl TSH enthaltendem Humanserum und 100 µl des radioaktiv markierten zweiten Antikörpers unter Schütteln bei Raumtemperatur wird die von den immobilisierten anti-hTSH-Antikörpern gebundene Radioaktivitätsmenge in einem Gammacounter gemessen. Die von den stabilisierten anti-hTSH-Antikörpern auf der Röhrchenoberfläche gebundene Menge an Radioaktivität ist der Menge an gebundenem TSH direkt proportional.

Die Präzision des Antigen-Bindungsverhaltens der stabilisierten Antikörper wird durch Bestimmung des Variationskoeffizienten VK (%) rechnerisch nach der folgenden Beziehung ermittelt:
VK = s/n x 100, worin s = Standardabweichung, n = Anzahl der Bestimmungen.

Beispiel 2

Die nach Beispiel 1a) immobilisierten Antikörper werden mit einer Lösung aus 7,5 kg eines Zuckeralkohol-Gemischs in Form des Handelsprodukts Karion (Wz) FP und 7,5 g Natriumazid in 150 l destilliertem Wasser zur Stabilisierung behandelt.

Die Lösung verbleibt zur Stabilisierung 30 min in den Röhrchen. Die Trocknung erfolgt durch Lyophilisation.

Beispiel 3

Die Stabilisierung der gemäß 1a) immobilisierten Antikörper erfolgt mit einem Gemisch aus 4,5 kg eines Zuckeralkoholgemischs in Forms des Handelsprodukts Karion (Wz) F, 10 l einer 3%igen gepufferten und sterilfiltrierten Lösung aus Eialbumin und 7,5 g Natriumazid in 150 l destilliertem Wasser.

Die Röhrchen werden mit jeweils 2 mal 5 ml dieser Lösung unter Verwendung einer automatischen Waschvorrichtung gewaschen. Die Dauer eines Waschvorgangs beträgt 1 min. Anschließend werden die Röhrchen lyophil oder an der Luft getrocknet.

Beispiel 4 (mit Vergleichen)

Die auf Polystyrol-Röhrchen adsorptiv immobilisierten monoklonalen anti-hTSH-Antikörper wurden mit verschiedenen, in der nachfolgenden Tabelle 1 angegebenen Stabilisatoren in Form ihrer wäßrigen Lösungen behandelt und anschließend durch Lyophilisation getrocknet. Danach wurde die Aktivität der erhaltenen Produkte in einem immunoradiometrischen Assay nach Zeiträumen von 1, 2, 4, 8 und 16 Wochen bestimmt.

Die Ergebnisse sind in der nachfolgenden Tabelle 1 zusammengefaßt.

Tab. 1: Aktivitaetserhalt von immobilisierten anti-hTSH-Antikoerpern nach vergleichender Behandlung mit Karion F und anderen bekannten Stabilisatoren (Anfangsaktivitaet = 100 %)

| Aktivitäts-erhalt | Wochen | | | | |
|---|---|---|---|---|---|
| Stabilisator | 1 | 2 | 4 | 8 | 16 |
| Karion F | 100 | 100 | 99 | 99 | 97 |
| Sorbit | 95 | 80 | 77 | 68 | 60 |
| Glucose | 93 | 80 | 73 | 70 | 55 |
| Saccharose | 93 | 82 | 73 | 65 | 56 |
| Glycerin | 50 | 33 | 10 | – | – |

Ein beginnender Denaturierungsprozeß der immobilisierten Antikörper ist, noch bevor es zu einer meßbaren Änderung der Bindungsaktivität kommt, an einem zunehmenden Verlust an Präzision bei der immunologischen Reaktion zu erkennen. Aus dem Verlauf der über die Dauer der Aufbewahrung ermittelten Präzision der Antikörper-hTSH-Reaktion, ausgedrückt als Variationskoeffizient VK (%), läßt sich daher der Einfluß und die Güte der stabilisierenden Substanzen ermitteln. Tabelle 2 enthält die Ergebnisse der Untersuchung der stabilisierenden Wirkung anhand von Veränderungen des Variationskoeffizienten VK (%) nach 0, 4, 8, 16, 32 und 48 Wochen.

```
Tab. 2:     Langzeit-Praezision (VK %) von
TSH-Antikoerpern beschichteten Polystyrol-Roehrchen
nach Behandlung mit Karion F und anderen bekannten
Stabilisatoren
```

| Präzision (VK %, n=10) | Wochen | | | | | |
|---|---|---|---|---|---|---|
| Stabilisator | 0 | 4 | 8 | 16 | 32 | 48 |
| Karion F | 1,6 | 2,0 | 2,3 | 2,3 | 2,7 | 2,7 |
| Saccharose | 3,2 | 4,1 | 5,1 | 5,8 | | |
| Glucose | 2,9 | 3,8 | 5,3 | 6,4 | | |
| Glycerin | 12,4 | 20,6 | – | – | | |

Die in den Tabellen 1 und 2 zusammengefaßten Untersuchungsergebnisse belegen eindeutig, daß die Stabilisierungswirkung eines erfindungsgemäß zu verwendenden Gemischs diejenige von Saccharose, Glucose und Glycerin sowie von Sorbit allein erheblich übertrifft.

Beispiel 5

Der Einfluß der Feststoffkonzentration des Gemischs aus Zuckeralkohol und hydrierten Oligosacchariden auf die Stabilisierung wurde unter Verwendung von Röhrchen gemäß den Beispielen 1 bis 3 anhand der Veränderung des Variationskoeffizienten wie in den vorausgehenden Beispielen bestimmt.
Es wurden die in Tabelle 3 zusammengefaßten Ergebnisse erhalten.

Tab. 3: Einfluß der Karion F - Konzentration auf die
Präzision der TSH-Antikörper beschichteten
Polystyrol-Röhrchen

| Karion F-Konzentration (Gew.-%) | Präzision (VK %, n=10) |
|---|---|
| 0 | 12,3 |
| 1 | 3,1 |
| 2 | 2,7 |
| 3 | 1,6 |
| 5 | 1,6 |
| 10 | 2,0 |

Es zeigt sich, daß Feststoffkonzentrationen von 1 bis 5 Gew.-%, insbesondere von etwa 3 Gew.-%, besonders gute Ergebnisse liefern.

Weitergehende Versuche zeigten, daß das erfindungsgemäße Verfahren nicht nur speziell zur Stabilisierung von anti-hTSH-Antikörpern wirksam ist, sondern daß ähnlich vorteilhafte Ergebnisse auch im Falle von anti-T3-Antikörpern, anti-T4-Antikörper, anti-PTH-Antikörpern und anti-Thyreoglobulin-Antikörpern sowie auch im Falle von immobilisierten T4- und T3-Antigenen erhalten werden.

## Patentansprüche

1. Verfahren zur Stabilisierung von biologisch aktiven Substanzen in immobilisierter Form, bei dem die immobilisierten biologisch aktiven Substanzen in Gegenwart eines inerten Füllstoffs getrocknet werden, dadurch gekennzeichnet, daß als Füllstoff ein Gemisch aus einem oder mehreren Zuckeralkoholen der Formel $CH_2OH(CHOH)_nCH_2OH$,
worin n eine ganze Zahl von 2 bis 4 ist,
und einem kristallisationsverzögernd wirkenden Anteil an hydrierten Oligosacchariden oder Zuckeralkohol-Derivaten verwendet wird.

2. Verfahren nach Anspruch 1, dadurch gekennzeichnet, daß der Zuckeralkohol D-Sorbit ist und 50 bis 95 Gew.-% des Feststoffgehalts des in Form einer wäßrigen Lösung eingesetzten Gemischs bildet.

3. Verfahren nach Anspruch 1 oder 2, dadurch gekennzeichnet, daß das Gemisch aus Zuckeralkohol(en) und Kristallisationsverzögerern in Form einer wäßrigen Lösung mit einer Feststoff-Konzentration von 0,1 bis 10 Gew.-%, vorzugsweise 1 bis 5 Gew.-%, auf die immobilisierten biologisch aktiven Substanzen aufgebracht und danach durch Lufttrocknung, Vakuumtrocknung oder Gefriertrocknung aufgetrocknet wird.

4. Verfahren nach einem der Ansprüche 1 bis 3, dadurch gekennzeichnet, daß die immobilisierten biologisch aktiven Substanzen auf Mikrotiterplatten oder an der Innenwand eines Kunststoffröhrchens adsorbiert und dadurch immobilisiert vorliegen (Coated tube-Technik).

5. Verfahren nach einem der Ansprüche 1 bis 4, dadurch gekennzeichnet, daß die immobilisierte biologisch aktive Substanz ein mono- oder polyklonaler Antikörper oder ein Hapten oder ein Antigen ist.

6. Verfahren nach Anspruch 5, dadurch gekennzeichnet, daß die immobilisierte biologisch aktive Substanz ein mono- oder polyklonaler anti-human Thyreotropin-Antikörper (hTSH-Ak) ist.

7. Verfahren nach einem der Ansprüche 1 bis 6, dadurch gekennzeichnet, daß die immobilisierten biologisch aktiven Substanzen zum Aufbringen des Gemischs aus Zuckeralkohol und Kristallisationsverzögerern mit einer wäßrigen Lösung dieses Gemischs ein- oder mehrmals gewaschen oder in eine solche Lösung eingetaucht werden, worauf man die Lösung 30 bis 120 min auf die immobilisierten biologisch aktiven Substanzen einwirken läßt.

## Claims

1. Process for the stabilisation of biologically active substances in immobilised form, in which the immobilised biologically active substances are dried in the presence of an inert filler, characterized in that the filler used comprises a mixture of one or more sugar alcohols of the formula $CH_2OH(CHOH)_nCH_2OH$, in which n is an integer from 2 to 4, and, in a proportion sufficient to retard crystallisation, of hydrogenated oligosaccharides or sugar alcohol derivatives.

2. Process according to Claim 1, characterized in that the sugar alcohol is D-sorbitol and forms 50 to 95 % by weight of the solids content of the mixture which is employed in the form of an aqueous solution.

3. Process according to Claim 1 or 2, characterized in that the mixture of sugar alcohol(s) and crystallisation retarders is applied to the immobilised biologically active substances in the form of an aqueous solution having a solids content of 0.1 to 10 % by weight, preferably 1 to 5 % by weight, and is then dried on by air-drying, vacuum-drying or freeze-drying.

4. Process according to any of Claims 1 to 3, characterized in that the immobilised biologically active substances are adsorbed, and thus immobilised, on microtitre plates or on the inner wall of a plastic tube (coated-tube technique).

5. Process according to any of Claims 1 to 4, characterized in that the immobilised biologically active substance is a mono- or polyclonal antibody or a hapten or an antigen.

6. Process according to Claim 5, characterized in that the immobilised biologically active substance is a mono- or polyclonal anti-human thyrotropin antibody (hTSH Ab).

7. Process according to any of Claims 1 to 6, characterized in that the immobilised biologically active substances are, for the application of the mixture of sugar alcohol and crystallisation retarders, washed one or more times with an aqueous solution cf this mixture, or immersed in such a solution, after which the solution is allowed to act on the immobilised biologically active substances for 30 to 120 min.

## Revendications

1. Procédé de stabilisation de substances biologiquement actives sous forme immobilisée, dans lequel les substances biologiquement actives immobilisées sont séchées en présence d'une matière de remplissage inerte, caractérisé en ce qu'un mélange constitué d'un ou de plusieurs alcools de sucre de formule $CH_2OH(CHOH)_nCH_2OH$, où n est un nombre entier compris entre 2 et 4, et d'une certaine proportion agissant comme retardateur de cristallisation, d'oligosaccharides hydratés ou de dérivés d'alcools de sucre, est utilisé comme matière de remplissage.

2. Procédé selon la revendication 1, caractérisé en ce que l'alcool de sucre est le D-sorbitol et constitue 50 à 95% en poids de la teneur en matière solide du mélange utilisé sous la forme d'une solution aqueuse.

3. Procédé selon la revendication 1 ou 2, caractérisé en ce que le mélange, constitué d'un ou de plusieurs alcools de sucre et d'agents retardateurs de cristallisation, est appliqué sur les substances biologiquement actives immobilisées sous la forme d'une solution aqueuse ayant une concentration en matière solide comprise entre 0,1 et 10% en poids, de préférence entre 1 et 5% en poids, et est ensuite séché par séchage à l'air, séchage à vide ou lyophilisation.

4.  Procédé selon l'une quelconque des revendications 1 à 3, caractérisé en ce que les substances biologiquement actives immobilisées sont présentes sous forme adsorbée et donc immobilisée sur des plaques de microtitrage ou sur la paroi intérieure d'un tube en plastique (technique dite "coated-tube").

5.  Procédé selon l'une quelconque des revendications 1 à 4, caractérisé en ce que la substance biologiquement active immobilisée est un anticorps monoclonal ou polyclonal ou un haptène ou un antigène.

6.  Procédé selon la revendication 5, caractérisé en ce que la substance biologiquement active immobilisée est un anticorps monoclonal ou polyclonal anti-thyrotrophine humaine (hTSH-Ac).

7.  Procédé selon l'une quelconque des revendications 1 à 6, caractérisé en ce que, pour appliquer le mélange constitué d'alcool de sucre et d'agents retardateurs de cristallisation, on lave les substances biologiquement actives immobilisées une ou plusieurs fois avec une solution aqueuse de ce mélange ou on les plonge dans une telle solution, après quoi on laisse agir la solution sur les substances biologiquement actives immobilisées pendant 30 à 120 min .